# EUROPEAN PATENT APPLICATION

(11) **EP 3 501 378 A1**
(43) Date of publication of application: **26.06.2019**
(21) Application number: 17209970.7
(22) Date of filing: 22.12.2017
(51) Int. Cl.: A61B 5/00, A61B 5/053, A61B 5/103

(54) **MOUNTING DEVICE, MOUNTING DEVICE TEMPLATE, DETECTION SYSTEM AMD METHOD OF USE THEREOF**

(71) Applicant: Koninklijke Philips N.V., 5656 AE Eindhoven (NL)
(72) Inventor: BEIJENS, Linda Goverdina Maria, 5656 AE Eindhoven (NL); Martin JURNA, Martin JURNA, 5656 AE Eindhoven (NL); VAN VLIMMEREN, Marijke Antonia Adriana, 5656 AE Eindhoven (NL); VAN GROOTEL-RENSEN, Maria Angelina Josepha, 5656 AE Eindhoven (NL); VERKRUIJSSE, Willem, 5656 AE Eindhoven (NL)
(74) Representative: de Haan, Poul Erik

(57) **Abstract**

The present invention relates to a mounting device enabling a user to determine the development of his or her skin condition with a detection system, a mounting device template, a method for use with a mounting device and a computer program.

## Description

### FIELD OF THE INVENTION

The present invention relates to a mounting device for skin monitoring, a mounting device template, a method for use with a mounting device, a detection system and a computer program.

### BACKGROUND OF THE INVENTION

Today's skin care market offers a wide range of products having a positive effect on facial skin features like skin smoothness, fine lines, and pigmentation. In clinical studies the effects of skin care products, in particular skin treatment devices, have been proven using high-end measurement equipment including photo booths with special and controlled lighting conditions (e.g. Visia-CR by Canfield).

Despite the clinically proven visible results users are often not able to see the treatment results at home as the results are subtle and appear gradually over time. This leads to limited consumer satisfaction. It was found, however, that consumer satisfaction can be increased when users can compare before- and after-treatment photographs themselves to visualize the effect.
There is also an increasing trend in self measurement. Skin, being the largest interface between the body and the outside world, plays a central role for this trend and provides a rich source of data. It is generally desired to improve people's lifestyle through interventions that are expressed/measurable on skin. The appearance of skin features such as eye bags or pimples and certain skin chromophores, e.g. hemoglobin or carotenoids, are closely linked to lifestyle habits and appearance and important aspects for both the male and female beauty business.

### SUMMARY OF THE INVENTION

It is an object of the present invention to provide a solution allowing monitoring of skin treatment at home.

In a first aspect of the present invention a mounting device is presented, which enables a user to detect skin features with a sensor unit, the mounting device comprising
one or more holding parts configured to hold the sensor unit,
one or more apertures configured to be aligned with a sensor of the sensor unit, and
one or more support elements configured to fix the mounting device at a predefined position of a body part of the user,
wherein the mounting device is configured to be made from foldable material.

In a second aspect of the present invention a mounting device template is presented, which is used for constructing a mounting device, the mounting device template comprising
one or more holding part templates configured to be arranged into one or more holding parts for holding a sensor unit,
one or more aperture templates configured to be arranged into one or more apertures to be aligned with the sensor unit, and
one or more support element templates configured to be arranged into one or more support elements for fixing the mounting device at a predefined position of a body part of the user, wherein the mounting device template is configured to be made from foldable material.

In another aspect of the present invention a method for use with a mounting device is presented, the method comprising
detecting a skin feature signal corresponding to a skin feature with a sensor unit using a mounting device at two or more different points in time,
storing skin feature data corresponding to the detected signal, and
determining development of the user's skin condition based on comparing the skin feature data of different points in time with each other.

In still another aspect of the present invention a detection system for use with a mounting device is presented, the system comprising
a sensor unit configured to detect a skin feature signal corresponding to a skin feature at two or more different points in time,
a storage unit configured to store skin feature data corresponding to the detected signal,
a comparing unit configured to compare the skin feature data of different points in time with each other and to determine development of the user's skin condition based on comparing the skin feature data.

In yet further aspects of the present invention, there are provided a computer program, for example a software application (e.g. a smartphone app), which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed mounting device template, method, detection system, computer program and medium have similar and/or identical preferred embodiments as the claimed mounting device, in particular as defined in the dependent claims and as disclosed herein.

The present invention is based on the idea to provide a mounting device allowing monitoring of skin treatment at home. Generally, monitoring of skin and particularly skin treatment effects at home is difficult for consumers. Nowadays nearly all consumers have smartphones to take photographs of themselves, but there are several limiting factors to the value of these photographs with respect to comparing the effectiveness of skin treatment outcomes or lifestyle changes over time. First of all, lighting conditions may differ enormously between two subsequent photo moments. In fact, light distribution of a smartphone is mostly not very suitable for visualizing specific skin conditions. Second, the positioning of a particular part of the skin is usually not standardized in home use. Hence, it is not guaranteed the same skin areas are compared which may lead to a wrong interpretation of the skin treatment progress. Furthermore, visualizing color changes or fine lines typically requires the use of special filters. Even upon using special skin sensors measuring skin moisture, for example, treatment effects can often not be tracked reliably, since users may not be capable to measure exactly the same skin parts over and over again.

To create comparable measurements of user's skin and to see the difference in skin condition over time, it is inevitable that the portion of skin used for measurements/detections is the same in each measurement. Furthermore, to generate comparable measurements of the user's skin environmental conditions should not differ between photo shootings. If the user's skin is analyzed by taking photos, for example, light conditions and flash adjustments should be the same for each photograph. To achieve this, there is provided a mounting device helping users to find repeatedly the correct position for skin detection.

The suggested mounting device is supposed to be worn or placed on a body part of the user and to be fixed with support elements. Since particularly monitoring of facial skin is important for users, support elements may comprise ear support elements and nose support elements, for example. Other support elements like straps or adhesive are conceivable as well. Apart from stably fixing the mounting device on a body part of the user the device also allows to hold a sensor unit in a fixed predefined position with a holding part. The holding part may either be constructed to hold the sensor unit such that it has direct skin contact or it may hold the sensor unit according to a predefined distance between skin and sensor. The holding part may be a container, a sachet, an elastic band or suction cup, for example. Furthermore, the holding part has to be placed such that its position with respect to an aperture is fixed.

Quite apart from the fact that monitoring skin treatment effects at home is not reliable so far or otherwise elaborate, there is the general problem that packaging material of skin treatment products is often only used to protect its content or to communicate with the consumer and carelessly thrown away after purchase. The suggested mounting device can be made from any foldable material and thus provides a sustainable solution. The material shall have a minimum stability to meet the above mentioned criteria, in particular to hold a sensor unit in a stable position.

Although the mounting device can be applied to any skin area of the user, the mounting device preferably is a head mounting device configured to be mounted on the head of the user. Of all skin areas facial skin is the most exposed to environmental influences and hence subject to significant changes with respect to wrinkles, pimples, skin moisture, sebum etc. On the other hand, skin care products are by far most applied to facial skin. In fact, the face usually is the first body part recognized by others and hence an important communication tool. Accordingly, most users are particularly interested in the development of skin condition with respect to their face.

Irrespective of the body region where the mounting device is applied, the device may be configured to adapt to the respective body region shape. In fact, the device may be configured to be placed tightly on the skin to ensure that external environmental light influences are minimized.
The user may obtain the mounting device in a prefabricated manner. However, the user may obtain said device also in the form of a template which is configured to be constructed as mounting device. In particular, the one or more holding part templates are configured to be arranged as one or more holding parts. For instance, a holding part template may be a click-out portion of a cardboard which upon clicking out becomes a holding part for the mounting device. Likewise, an aperture template is configured to be arranged as aperture and a support element template is configured to be arranged as support element. Conversion of mounting device templates into units of the mounting device may be achieved, for example, by clicking out, cutting out or by folding predefined pieces of an appropriate material such as paper, cardboard or particular kinds of plastic. To aid construction of the mounting device, template parts may comprise crease lines and pre-formed creases and one or more cuts and flaps configured to be put into said cuts or adhesive, in particular adhesive applied on flaps. Apart from facilitating construction these features may also improve the stability and longevity of the mounting device.

The present invention further presents a corresponding detection system configured to detect a skin feature suited for analyzing the user's skin condition over time. The detection system may comprise the sensor unit, storage unit and comprising unit in a single device or in a distributed manner. The detection system may be a smartphone of the user, for example, comprising next to a photo-camera configured to be used as sensor unit an application for image storing and easy comparison of pre- and after skin treatment photographs. However, the sensor unit may also be located in a different device than the other units. The sensor unit may be a contact sensor, for example, specially designed to detect wrinkles and the data of skin detection may be transmitted from the sensor unit to a laptop or PC located elsewhere. Data storage and evaluation of data may then be performed by said laptop or PC.

In general, color, contrast and their location and spread can be used to detect and compare particular skin features like spots and pimples, for example. Apart from taking photos or other skin detections as snapshot the sensor unit may also be capable of performing a long-term measurement, i.e. to detect a skin feature signal corresponding to a skin feature in temporally different detection sessions. In particular, videos of the user's skin may be taken which e.g. allows to perform a photoplethysmography (PPG) for quantification of skin radiance. In case of long-term measurements the comparing unit is configured to compare the skin feature data of different measurement sessions with each other.

In an embodiment of the mounting device, the device further comprises one or more cuts, cut-outs, sketched cut-outs, pre-perforated click-out portions and/or foldable portions configured to fix the body part of the user in the predefined position and/or to attach any one of light elements, light filters, polarization filters and color filters. Several different cut-out sketches and/or pre-perforated click-out portions may be used to create support elements, optional recess areas, and/or apertures of different size. Since the shape and size of sensor units and possibly corresponding devices may differ enormously, optical recess areas that can be created using foldable portions, for example, allow perfect fitting of said units and devices in the mounting device. Likewise creating adaptable apertures via click-out portions helps adapting the mounting device to different smartphone lenses. Apart from that, several click-out parts also provide the possibility to perform skin measurements at different places at the same time. Foldable portions are particularly helpful to be used as ear support elements, chin support elements or forehead supports. In general, they may be used to adapt the device to the skin contour or body shape of the user. Hence, click-out portions and foldable potions may also be used to adapt the light conditions inside the mounting device, in particular to minimize impacts caused by changes of environmental light conditions.

In another embodiment, the mounting device is a portion of one of a mailer, a package insert, a user manual, a leaflet, a product packaging, in particular the product packaging of a skin care product, a carton, a box lid, or a shipping box. On the one hand, reusing material implies low production costs of the mounting device. On the other hand, such material is environment-friendly and sustainable. This particularly applies if the material, e.g. a packaging box used, is made of recyclable material.

Preferably, the mounting device comprises a calibration bar configured to facilitate calibration of the sensor unit. The calibration bar may be a color bar used to calibrate the camera of a smartphone, for example. However, the calibration bar may also be configured to be used for sensor units working in the invisible spectrum or for moisture sensors. Apart from that, it can be used to adjust the flashlight of a camera. The calibration bar may be a bar in the proper meaning of the word but may also be calibration table or calibration scale.

In another embodiment, the mounting device comprises one or more crease lines and/or pre-formed creases and/or one or more positioning marks configured to aid positioning of the body part in the predefined position. Crease lines and pre-formed creases can be used to indicate flaps and to allow easier repositioning of flaps, respectively. Hence, creases indirectly facilitate influencing the adaption of light conditions by moving flaps. Positioning marks may be simple, possibly labelled lines, marking a position of a body part required to be in a particular area of the mounting device. Positioning marks may indicate a required position of the ears, eyes, nose and lips, for example. Positioning marks may also comprise pre-sketched face parts which guide the user in a self-explanatory manner to the required head position. Moreover, a positioning mark may be used for repositioning of device parts like holding parts. Still further, positioning marks can comprise distance sensors configured to measure the distance between skin and mounting device at the position of the distance sensor. The distance sensor may further be configured to transmit data corresponding to the measured distance to an indication unit indicating whether a predefined distance is complied with or not. Said indication unit may comprise light elements, for example, which light up as soon as the predefined position is reached.

In an advantageous embodiment, the foldable material of the mounting device any one of cardboard, paper, plastic, and textile and/or a part or the whole skin facing side of the mounting device is laminated, opaque, colored and/or comprises reflecting or absorbing structure. Cardboard, soft plastics and robust textiles offer the advantage to be flexible but still stable. Coloring and lamination allow for different color reflections and/or less or more light reflection from an illumination source towards the skin via a skin facing side of the mounting device. Different levels of opacity of the mounting device may also be used to influence the illumination of underlying skin. The higher the opacity the less (natural) light may reach the user's skin and the less are the illumination differences caused by illumination of the environment. Hence, it is usually preferred that the mounting device is made of highly opaque material. Different structures of material the mounting device is made of on (parts of) the skin facing side may likewise influence lighting conditions. While roughened surfaces may be used to absorb detection signals, smooth surfaces may be used to reflect said signals.

In a further embodiment of the mounting device, the device comprises one or more mirrors configured to reflect lighting, and/or one or more light elements configured to illuminate the skin of the user, and/or one or more light filters and/or polarization filters configured to generate and/or exclude specular reflectance and/or one or more color.

The mirrors may be mirrors for visible but as well for invisible light, such as light of the IR spectrum. They can be attached to any part of a skin facing side of the mounting device to redirect light of a camera's flash or other sensor signals. This allows various positions of the camera with respect to the skin. The mirrors could also be used to reflect detection signals such as lighting in a different direction than straight from the detection signal source such as a camera.

Next to the lighting elements that come with a detection system including the sensor unit such as the flashlight of a smartphone, for example, the mounting device may further include light elements of its own. The light elements can be any one of light emitting diodes (LEDs), in particular colored LEDs, incandescent light bulbs, halogen lamps and fluorescent lamps. Preferably, the light elements are operated by one or more batteries located in a battery compartment of the mounting device. They can be attached to any part of a skin facing side of the mounting device. Preferably, they are attached in the vicinity of an aperture or mirror. Further preferably, their light is directed to a part of the skin to be detected and to illuminate said part in a uniform manner. In particular, light elements of specific wavelengths may be used to enhance certain skin features and to analyze skin chromophores.

Polarization filters are placed in front of an aperture of the mounting device to cover the lens of a camera in order to manage reflections or to suppress glare. Specular reflectance may be enhanced or reduced with said filters. Particularly wrinkle and skin texture measurement can be done using shadows created with parallel polarized lighting conditions. However, circular polarization filters may also be used.

Light filters may be used to illuminate the skin only with a specific wavelength range. For example, there can be applied a rotating filter window comprising one or more segments with different color filters to an aperture such that the user can put a desired color or a color required by their application in front of a camera lens.

In an embodiment of the method for use with a mounting device, the method further comprises checking body part position of the user in the mounting device, and providing feedback to the user on the body part position in the mounting device. Checking the body part position may be performed using distance detectors measuring the distance between the skin of the user and the mounting device at the respective detector positions with respect to a predefined distance threshold. Likewise, contact detectors may be used to detect contact of predefined parts of the mounting device with the skin of the user. Feedback concerning the required body part positioning may be provided by the mounting device itself or via other devices evaluating the detection signals of said detectors. The same applies to providing instructions for repositioning. These may be provided by an audible or visible signal. Instructions of positioning may be provided based on comparison with previous measurement positions or based on live measuring only. In the latter case, preset alignment conditions are specified and the user is instructed to move a respective body part, for example his head, until positioning conditions are met.

In another embodiment of the method for use with a mounting device, the method comprises transmitting skin feature data of different points in time to an expert for further assessment and feedback. Skin features data include photos of the skin and other measurement results, for example moisture level of the skin or an IR image of the skin. Transmitting may particularly be performed via the internet. For example, the user may take one or more photos at different points in time with his smartphone and upload said images or send said images using an appropriate smartphone application to a dermatologist.

In an embodiment of the detection system for use with a mounting device, the sensor unit is any one of a photo sensor configured to detect a photo of the skin in the visible or non-visible range, in particular in the IR-range, a moisture sensor configured to measure moisture of the skin, an impedance sensor configured to measure impedance of the skin, a conductance sensor configured to measure conductance of the skin, a pH sensor configured to measure the pH value of the skin, a sebum sensor configured to measure sebum of the skin and a microflora sensor configured to detect microflora of the skin, and/or the detection system further comprises a user interface configured to present the development of the user's skin to the user and/or a calibration unit configured to calibrate the sensor.

### BRIEF DESCRIPTION OF THE DRAWINGS

These and other aspects of the invention will be apparent from and elucidated with reference to the embodiment(s) described hereinafter. In the following drawings
Fig. 1 shows a perspective view of a first embodiment of a mounting device according to the present invention,
Fig. 2 shows a perspective view of a second embodiment of a mounting device according to the present invention,
Fig. 3 shows a perspective view of a third embodiment of a mounting device according to the present invention,
Fig. 4 shows a perspective view of a fourth embodiment of a mounting device according to the present invention,
Fig. 5 shows a top view of a first embodiment of a mounting device template according to the present invention,
Fig. 6 shows a side view of an embodiment of a mounting device and of an embodiment of a detection system in the form of a detection device according to the present invention,
Fig. 7 shows a schematic diagram of a first embodiment of a detection system in the form of a detection device according to the present invention, and
Fig. 8 shows a schematic diagram of a second embodiment of a detection system in the form of a detection device according to the present invention.
Fig. 9 shows a schematic diagram of a third embodiment of a detection system according to the present invention.

### DETAILED DESCRIPTION OF THE INVENTION

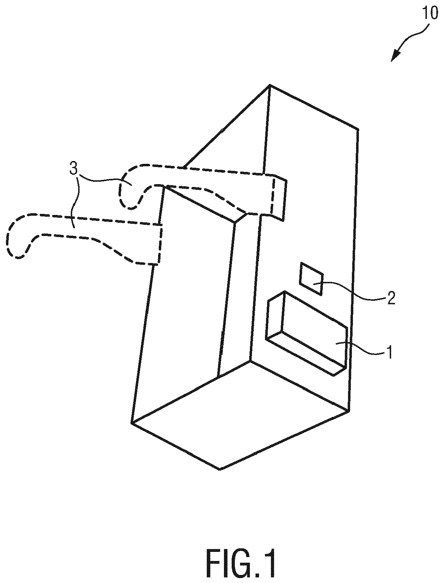

Fig. 1 shows a perspective view of a first embodiment of a mounting device 10 according to the present invention. This embodiment is particularly configured for facial skin examination. The mounting device 10 comprises a holding part 1, an aperture 2 and two support elements 3. The mounting device is made of a packaging box lid, wherein the holding part 1 and aperture 2 are arranged on a side part of said lid. While the aperture 2 is represented by a rectangular cut-out, the holding part 1 is a glued-on part. More specifically, the holding part 1 is a container whose size is configured to fit a particular sensor unit such as to hold the sensor unit in an unshakeable manner. The support elements 3 are glued-on parts as well. As ear support elements they are foldable parts of the mounting device 1. Their shape is adapted to the shape of ears of a user.

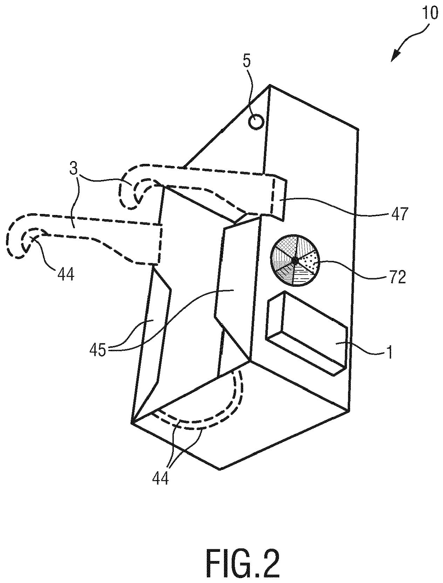

Fig. 2 shows a perspective view of a second embodiment of a mounting device 10 according to the present invention. In this embodiment, the mounting device 10 comprises a holding part 1, two ear support elements 3, a light element 5 and several foldable parts 45 and pre-perforated click-out portions 44. The ear support holders 3 are configured to fix the device on the head of a user. Hence, this embodiment is intended to monitor the skin of a user's face. The mounting device further comprises an aperture which is covered by a polarization filter 72. In this embodiment, the polarization filter 72 is configured to be a rotatable disc comprising several segments, wherein the different segments comprise different polarization filters. For example, one segment may comprise a circular polarization filter whereas the other segments may comprise different linear polarization filters. The size of filter segments is adapted to the size of the aperture 2 so that a segment may cover the aperture 2 completely. The light element 5 and the two foldable parts 45 may be used to adapt the illumination inside the mounting device 10, i.e. on the skin facing side of the device. In this embodiment, the light element 5 is a LED configured to increase the illumination of the skin, the foldable portions 45 are used to exclude ambient light of the environment. The pre-perforated click-out parts 44 may be clicked out to achieve a better adaption of the mounting device 10 to the ear and face contours of the user.

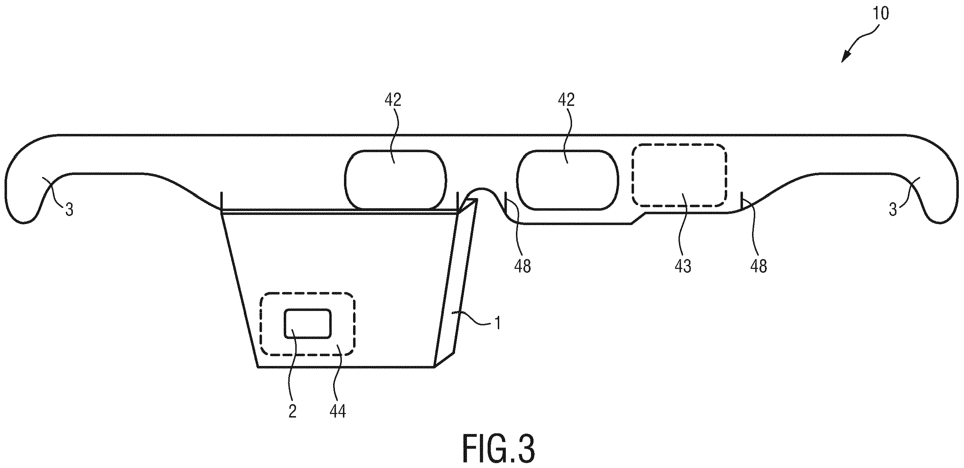

Fig. 3 shows a perspective view of a third embodiment of a mounting device 10 according to the present invention. This embodiment is configured to determine the condition of skin of a user's face, in particular to examine the condition of skin on a cheek. The shape of the device 10 is adapted to the head contours of the user to prevent irritations.

The mounting device 10 comprises two foldable temples 3, an aperture 2 and a container like holder 1 for holding a sensor unit comprised within a detection system in the form of a detection device 200. The holding part 1 is attached to a part of the mounting device 10 with removable adhesive 90. This allows to relocate the holder 1 to other parts of the device 10. Positions marks 48 printed on the device 10 allow precise (re)location of the holder. Furthermore, to ensure compatibility with a wide range of skin sensors 210 the holding part 1 comprises a pre-perforated click-out part 44 which allows enlarging the aperture 2. Cut-outs 42 are placed such that the eyes of the user are not occluded by the device 10. An additional sketched cut-out 43 may be cut out by the user to get a second aperture 2 in the user's temple region.

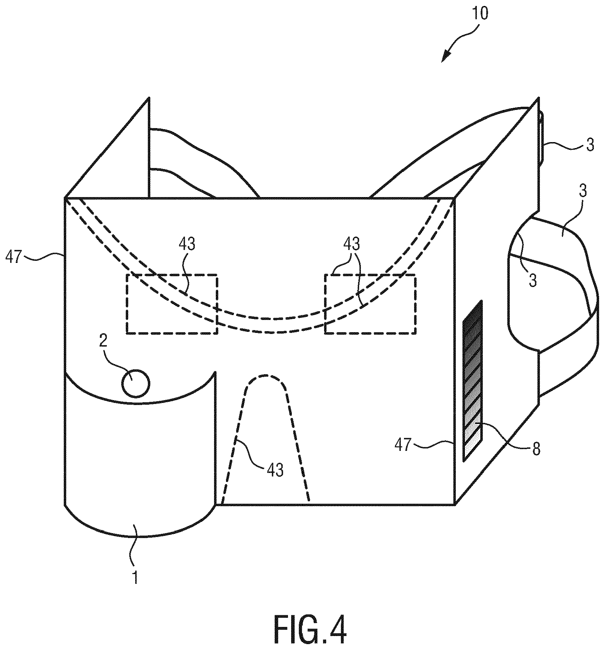

Fig. 4 shows a perspective view of a fourth embodiment of a mounting device 10 according to the present invention. This embodiment may be mounted on the head of a user but also may be mounted on other body parts such as the neck, for example. The device 10 comprises three sides which are interconnected by pre-formed creases 47. Thus the user may adapt the position among the three sides, i.e. change their angles to each other, in order to fit a desired body part. The device 10 can be mounted stably on the body using adjustable straps 3. Cutting out the sketched cut-outs 43 and using said cut-outs as support elements 3 may further help to fix the device 10 stably on the user's body. The device 10, for example, comprises a pre-sketched nose cut-out which may be cut out to generate a further support element 3. The device 10 further comprises a circular aperture 2 and a holding part 1 which is represented by an elastic cord attached to the skin averted side of the device 10. The elastic cord is configured to hold tightly a detection device 200 comprising a sensor unit 210. In order to calibrate the sensor unit 210 of the detection device 200 there is printed on a calibration bar 8 on the mounting device 10.

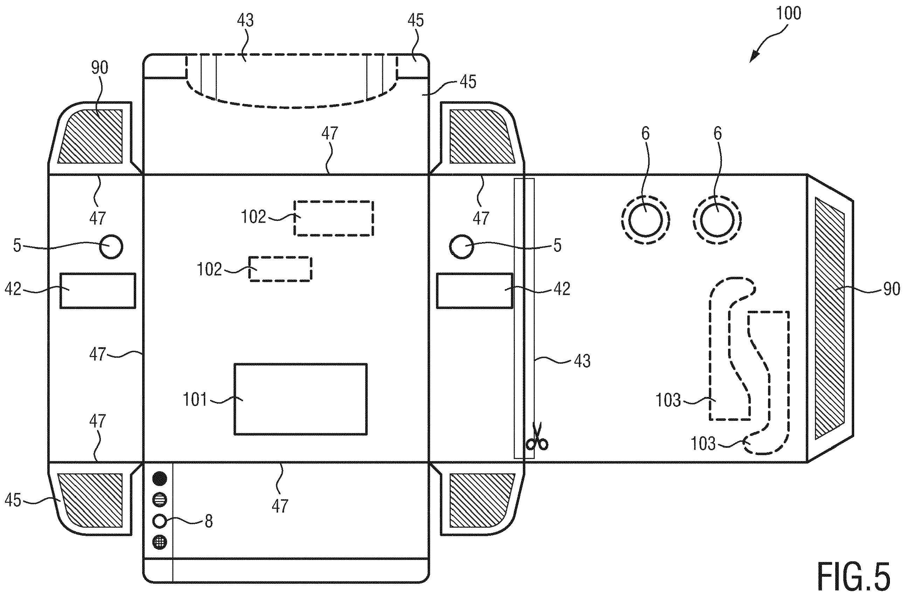

Fig. 5 shows a top view of a first embodiment of a mounting device template 100 according to the present invention. The mounting device template 100 is made from a fold-out packaging of a skin care product. It comprises a holding part template 101, which is a cut-out part 42, two aperture templates 102 and two support elements. In this embodiment, the holding part template 102 is a cut-out part and the aperture templates 102 are pre-perforated click-out portions. To support construction of a mounting device 10, the template 100 comprises several pre-formed creases 47 and sketched cut-outs 43. Furthermore, several flaps 45 of the template comprise adhesive 90 to ensure longevity of the mounting device 10 to be constructed. Upon delivery the adhesive may be covered by a cover sheet to prevent drying out of the adhesive. The mounting device template 100 further comes with two integrated, battery operated LEDs to illuminate the skin and two click-out mirrors 6 configured to be attached to the mounting device 10 for reflecting light of the light elements 5 and/or flashlight of a camera. This is particularly helpful in case the sensor unit 210 is a photo sensor for skin detection. A calibration bar 8 also included in the template allows calibration of the sensor unit 210 such that comparable photos of the user's skin can be taken repeatedly.

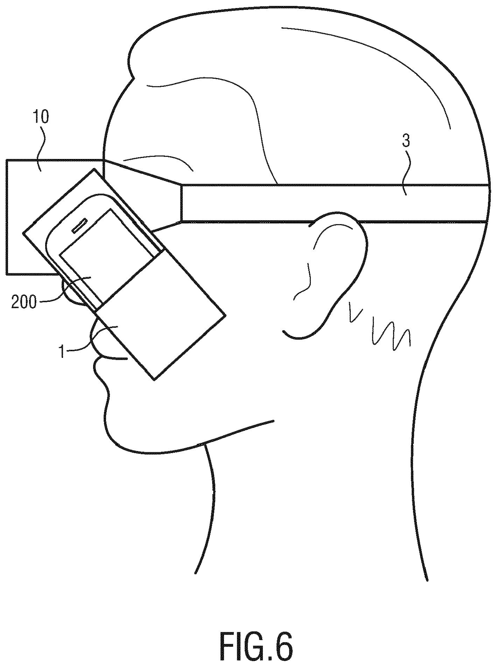

Fig. 6 shows a side view of an embodiment of a mounting device 10 and of an embodiment of a detection system in the form of a detection device 200 according to the present invention. The mounting device 10 comprises a holding part 1 holding the detection device 200 comprising a sensor unit 210 and a support element 3 in the form of a strap around the user's head. The aperture(s) of the mounting device 10 are covered by the detection device 200 in this Fig.

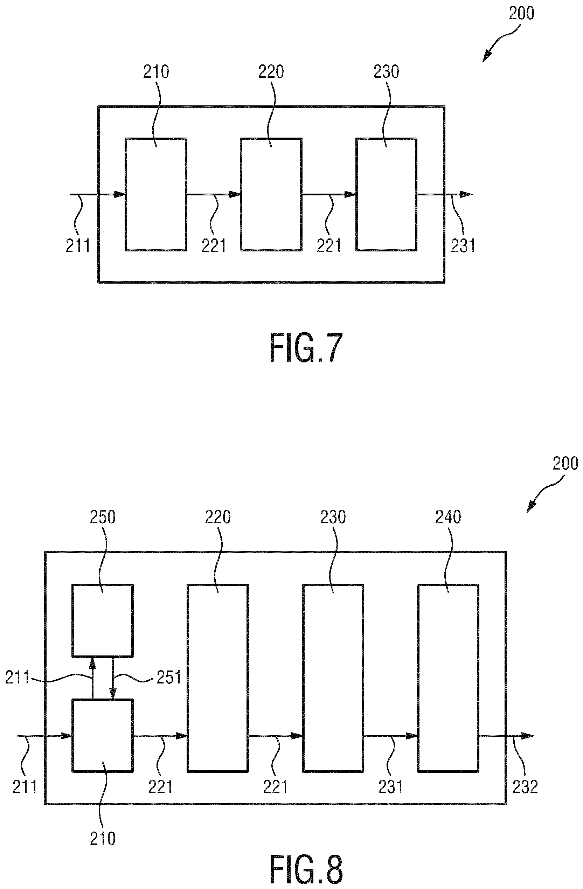

Fig. 7 shows a schematic diagram of a first embodiment of a detection system in the form of a detection device 200 according to the present invention. The device 200 includes a sensor unit 210, a storage unit 220 and a comparing unit 230. Upon receiving a sensor signal 211, i.e. a skin feature signal 211, the sensor 210 extracts skin feature data 221 and transmits said data 221 to the storage unit 220. In this embodiment, the sensor unit 210 is an impedance sensor measuring the impedance of the skin. Hence, after receiving an impedance signal of the user's skin, impedance data are extracted by the sensor and passed on to the storage unit 220. In the storage unit these data are stored and further passed on to the comparing unit 230. If there exist data corresponding to at least two impedance measurements of one and the same skin part corresponding to different points in time the comparing unit 230 compares the respective impedance values with each other. By analyzing the comparison result(s) 231 the comparing unit 230 may determine developments in the user's skin condition.

Fig. 8 shows a schematic diagram of a second embodiment of a detection system in the form of a detection device 200 according to the present invention. Apart from basic equipment this embodiment further comprises a user interface 240 and a calibration unit 250. The detection device 200 works in two different modes: a calibration mode and a skin feature detection mode. In the calibration mode the sensor unit 210 transmits a detected sensor signal 211 to the calibration unit 250. After receiving the sensor signal 211 the calibration unit generates a calibration signal 251 based on the sensor signal 211 and transmits said calibration signal 251 to the sensor 210 for calibration. Once the sensor unit 210 has been calibrated by the calibration signal the mode of the device 200 may be switched to the skin feature detection mode. In this mode the sensor 210 may obtain a detection signal 211 and generate skin feature data 221 corresponding to said signal 211. The skin feature data are then further processed in the storage unit 220 and comparing unit 230. The comparison result(s) 231 of the comparing unit are then transmitted to the user interface 240 which is configured to present the results to the user.

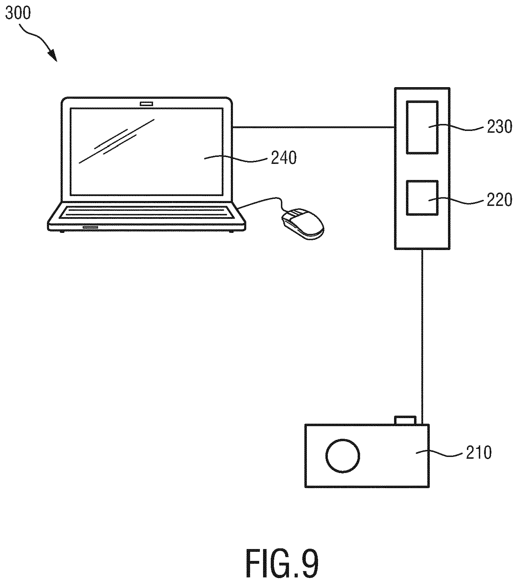

Fig. 9 shows a schematic diagram of a third embodiment of a detection system 300 according to the present invention. The system 300 comprises a sensor unit 210, a storage unit 220, a comparing unit 230 and a user interface 240. In this embodiment, the sensor unit 210 is a camera. The storage unit 220 and the comparing unit 230 are part of a personal computer and the user interface 240 is represented by a computer display.

After taking photos of one and the same skin part at different points in the time with his camera 210, the user transmits said photos to the storage unit 220 of a computer. The display 240 may then present the photos and the corresponding time of shooting to the user. The user may then decide which photos to compare and transmit said decision to the comparing unit 230 via the user interface 240. Subsequently, the comparing unit 230 compares photos with respect to said decision. The results of the comparison are then displayed on the display 240.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A mounting device (10) enabling a user to detect skin features with a sensor unit (210), the mounting device (10) comprising
one or more holding parts (1) configured to hold the sensor unit (210),
one or more apertures (2) configured to be aligned with the sensor unit (210), and
one or more support elements (3) configured to fix the mounting device (10) at a predefined position of a body part of the user,
wherein the mounting device (10) is configured to be made from foldable material.

2. The mounting device (10) according to claim 1,
further comprising one or more cut-outs (42), sketched cut-outs (43), pre-perforated click-out portions (44) and/or foldable portions (45) configured to fix the body part of the user in the predefined position and/or to attach any one of light elements (5), light filters (71), polarization filters (72) and color filters (73).

3. The mounting device (10) according to any one of the preceding claims, wherein the mounting device (10) is a portion of one of a mailer, a package insert, a user manual, a leaflet, a product packaging, in particular the product packaging of a skin care product, a carton, a box lid, or a shipping box.

4. The mounting device (10) according to any one of the preceding claims, further comprising a calibration bar (8) configured to facilitate calibration of the sensor unit (210).

5. The mounting device (10) according to any one of the preceding claims, further comprising one or more crease lines (46) and/or pre-formed creases (47) and/or one or more positioning marks (48) configured to aid positioning of the body part in the predefined position.

6. The mounting device (10) according to any one of the preceding claims, wherein the foldable material comprises any one of cardboard, paper, plastic, and textile, and/or wherein a part or the whole skin facing side is laminated, opaque, colored and/or comprises reflecting or absorbing structure.

7. The mounting device (10) according to any one of the preceding claims, further comprising one or more mirrors (6) configured to reflect lighting, and/or
one or more light elements (5) configured to illuminate the skin of the user, and/or one or more light filters (71) and/or polarization filters (72) configured to generate and/or exclude specular reflectance and/or one or more color filters.

8. A mounting device template (100) for constructing a mounting device (10) comprising
one or more holding part templates (101) configured to be arranged into one or more holding parts (1) for holding a sensor unit (210),
one or more aperture templates (102) configured to be arranged into one or more apertures to be aligned with a sensor unit (210), and
one or more support element templates (103) configured to be arranged into one or more support elements (3) for fixing the mounting device (10) at a predefined position of a body part of the user, wherein the mounting device template (100) is configured to be made from foldable material.

9. A method for use with a mounting device (10) comprising
detecting a skin feature signal (211) corresponding to a skin feature with a sensor unit (210) using a mounting device (10) at two or more different points in time,
storing skin feature data (221) corresponding to the detected signal (211), and
determining development of the user's skin condition based on comparing the skin feature data (221) of different points in time with each other.

10. The method according to claim 9, further comprising
checking body part position of the user in the mounting device (10), and
providing feedback to the user on the body part position in the mounting device (10).

11. The method according to claims 9 to 10, further comprising transmitting skin feature data (221) of different points in time to an expert for further assessment and feedback.

12. A computer program comprising program code means for causing a computer to carry out the steps of the method as claimed in claim 9 when said computer program is carried out on the computer.

13. A detection system (200, 300) for use with a mounting device, the device comprising
a sensor unit (210) configured to detect a skin feature signal (211) corresponding to a skin feature at two or more different points in time,
a storage unit (220) configured to store skin feature data (221) corresponding to the detected signal (211),
a comparing unit (230) configured to compare the skin feature data (221) of different points in time with each other and to determine development of the user's skin condition based on comparing the skin feature data (221).

14. The detection system according to claim 13,
wherein the sensor unit (210) is any one of a photo sensor configured to detect a photo of the skin in the visible or non-visible range, in particular in the IR-range, a moisture sensor configured to measure moisture of the skin, an impedance sensor configured to measure impedance of the skin, an conductance sensor configured to measure conductance of the skin, a pH sensor configured to measure the pH value of the skin, a sebum sensor configured to measure sebum of the skin and a microflora sensor configured to detect microflora of the skin.

15. The detection system according to claim 13,
further comprising a user interface (240) configured to present the development of the user's skin to the user and/or a calibration unit (250) configured to calibrate the sensor (210).
